# EUROPÄISCHE PATENTANMELDUNG

(11) **EP 1 295 538 A2**
(43) Veröffentlichungstag der Anmeldung: **26.03.2003**
(21) Anmeldenummer: 02020524.1
(22) Anmeldetag: 16.09.2002
(51) Int. Cl.: A23P 1/04, A23L 1/0526, A23K 1/00, A61K 47/36, A61K 9/50, A61K 7/00, A61K 7/48

(54) **Verkapselte Johannisbrotfasern mit verbesserten Produkteigenschaften, Verfahren zu ihrer Herstellung und ihre Anwendung**

(30) Priorität: 25.09.2001 DE 10147156
(71) Anmelder: Nutrinova Nutrition Specialties & Food Ingredients GmbH, 65929 Frankfurt am Main (DE)
(72) Erfinder: Haber, Bernd Dr., 55126 Mainz (DE); Ter Meer, Hans-Ulrich Dr., 60431 Frankfurt (DE)
(74) Vertreter: Schweitzer, Klaus, Dr.

(57) **Zusammenfassung**

Die Erfindung betrifft Ballaststoffkapseln, bestehend aus einem Kern aus im wesentlichen wasserunlöslicher Johannisbrotfaser und einem Kapselmaterial aus mindestens einem wasserlöslichen Ballaststoff. Weiterhin betrifft die Erfindung ein Verfahren zur Herstellung solcher Kapseln sowie deren Verwendung, insbesondere in Lebensmitteln, Tierfutter, Kosmetika und Arzneien.

## Beschreibung

Die Erfindung betrifft verkapselte Johannisbrotfasern, bestehend aus unlöslichen Johannisbrotfasern und einem oder mehreren Kapselmaterialien. Darüber hinaus betrifft sie sowohl den Herstellungsprozess als auch die Verwendung dieser stabilen Johannisbrotfaserkapsel.

Ballaststoffe sind für den Menschen unverdauliche Nahrungsbestandteile von Pflanzen. Sie umfassen unverdaubare Polysaccharide, Oligosaccharide, Lignin und assoziierte Substanzen. Ballaststoffe sind aus ernährungsphysiologischer Sicht sehr wichtig und können neben einem verdauungsfördernden Effekt auch regulierend auf Blutcholesterin und Blutglukosespiegel wirken. Darüber hinaus zeigen im Dickdarm fermentierbare Oligo- und Polysaccharide eine präbiotische Wirkung. Ballaststoffreiche Nahrung wird auch als Ursache für das relativ seltene Auftreten von Darmkrebs in Afrika oder bei bestimmten Bevölkerungsgruppen angegeben. In den meisten Industriestaaten ist dagegen die Aufnahme an Ballaststoffen deutlich unter der von Ernährungswissenschaftlern empfohlenen Tagesdosis von mindestens 30 g. Eine vermehrte Zufuhr an Ballaststoffen ist daher aus ernährungsphysiologischer Sicht wünschenswert. Dabei gilt es ein ausgewogenes Verhältnis zwischen löslichen und unlöslichen Ballaststoffen in der Ernährung zu erreichen.

Da sich diese Erkenntnisse immer weiter durchsetzen, wird der Wunsch des Verbrauchers nach Lebensmitteln mit einem speziellen Gesundheitsnutzen immer lauter. In der Praxis besteht bei Anreicherung von Lebensmitteln mit Ballaststoffen aber das Problem, dass dabei Textur und Mundgefühl sowie die Farbe leiden können.

Johannisbrot ist eine mediterrane Frucht, die reich an Kohlenhydraten, Ballaststoffen und Polyphenolen ist. Im Vergleich zu gängigen Ballaststoffquellen wie Getreide, Früchte und Gemüse ist der aus dem Fruchtfleisch des Johannisbrotes gewonnene unlösliche, in der Praxis Johannisbrotfaser genannte Ballaststoff von einer einzigartigen Zusammensetzung und zeigt neben seiner verdauungsfördernden Wirkung noch weitere gesundheitliche Zusatznutzen (Pérez-Olleros et al., 1999, J. Sci. Food Agric. 79, 173-178). In der EP-B-0 616 780 wird ein Herstellungsverfahren für ballaststoffreiche Präparate aus Johannisbrot beschrieben. Aufgrund des besonderen Herstellungsprozesses erhält man Präparate, die reich an unlöslichen Johannisbrotfasern sind.

Aufgrund seiner verschiedenen bioaktiven Bestandteile ist Johannisbrotfaser eine interessante Zutat zur funktionellen Anreicherung einer breiten Palette von Lebensmitteln. Aufgrund der Unlöslichkeit und der dunklen Färbung ist der Einsatz der Johannisbrotfaser allerdings auf bestimmte Lebensmittelgruppen beschränkt. Die dunkle Färbung macht sich bei von Natur aus helleren Lebensmitteln stärker bemerkbar. Außerdem zeigt sich bei Zusatz von Johannisbrotfaser oft eine deutliche sensorische Wahrnehmung der Partikel in der Lebensmittelmatrix ("kratzender Beigeschmack").

Die Technik der Mikroverkapselung wird in verschieden Bereichen (z.B. Kolloid- und Polymerchemie, Suspensions- und Trockentechnologie, pharmazeutische Technik, Lebensmitteltechnik) eingesetzt. Je nach Anwendungsgebiet stehen der Schutz des umhüllten Materials vor chemischen und/oder physikalischen Veränderungen oder die kontrollierte Freisetzung von Substanzen im Vordergrund. Des weiteren kann man aber auch Geruch, Geschmack oder ein negatives Mundgefühl des verkapselten Stoffs maskieren. Seit einiger Zeit wird die Mikroverkapselung immer stärker auch in der Lebensmitteltechnologie genutzt. Das Verfahren der Sprühtrocknung ist eines der am häufigsten angewendeten Verfahren zur Verkapselung verschiedener Substanzen in der Lebensmittelindustrie. Aber auch andere Umhüllungsverfahren wie das Fließbett/Wirbelbettverfahren oder der Wurster Prozess eignen sich für Lebensmittelzutaten wie Aromen, Gewürze, Fette und Fettsäuren, Vitamine, Farbstoffe usw.

In der US-A-4,619,831 wird von Ballaststoffpräparaten und deren Herstellungsprozess berichtet, die aus unlöslichen und löslichen Ballaststoffen gebildet werden. Dabei sind die unlöslichen Ballaststoffe mit einem dünnen Film von löslichen Ballaststoffen überzogen. Dies wird durch einfaches Mischen oder ein Überzugsverfahren erreicht.

Mischungen aus löslichen und unlöslichen Ballaststoffen sind auch in der EP-A-0 756 828 beschrieben. Die Kombination dient der Optimierung des ernährungsphysiologischen Nutzens von Ballaststoffen.

Mit Zein verkapselte Ballaststoffpartikel, insbesondere Guargummi, als Bestandteile von cholesterinsenkenden Lebensmitteln sind in der US-A-5,545,414 beschrieben. Guargummi ist dort von einer Zeinkapsel umgeben.

Die US-A-5,599,556 beschreibt Prolaminüberzüge, um unerwünschte Geschmackseindrücke zu maskieren. Es werden auch ernährungsphysiologische Stoffe wie Ballaststoffe genannt.

Als für Wasser "invisible fiber" wird eine Technologie beschrieben, bei der Ballaststoffe mittels Verkapselung mit Milchproteinen für einen breiteren Einsatz in Lebensmitteln zugänglich sind ("New Technology Boosts Fiber in Foods", C.I. Onwulata, Agricultural Research, March 2001, 14-15).

Die bisher beschriebenen Präparate aus löslichen Ballaststoffen und unlöslichen Ballaststoffen sind oft reine Mischpräparate ohne besondere Struktur oder stellen aufgrund zu geringer Stabilitäten keine befriedigende Lösung für einen breiteren Einsatz in Lebensmitteln dar. Insbesondere wären sie nicht geeignet, langfristig stabile dunkelgefärbte Ballaststoffe wie Johannisbrotfaser für einen breiten Einsatz in empfindlichen Lebensmitteln verfügbar zu machen. Aus ernährungsphysiologischer Sicht wäre besonders wünschenswert, ein Ballaststoffpräparat aus unlöslichen Ballaststoffen wie Johannisbrotfaser mit löslichen Ballaststoffen zu stabilisieren. Durch die Kombination der unterschiedlichen Wirkungen von löslichen und unlöslichen Ballaststoffen in Form einer Ballaststoffkapsel ließe sich neben der Verbesserung der Einsetzbarkeit des unlöslichen Ballaststoffs eine besonders gute Zutat erreichen, um Lebensmittel ernährungsphysiologisch ausgewogen mit Ballaststoffen zu ergänzen.

Aufgabe der vorliegenden Erfindung war es deshalb, ein Ballaststoffpräparat aus der ernährungsphysiologisch besonders interessanten Johannisbrotfaser zur Verfügung zu stellen, welches gleichzeitig
1. heller als Johannisbrotfaser selbst ist und dadurch farbliche Einflüsse von Johannisbrotfaser auf Lebensmittel bei Verarbeitung und Lagerung reduziert,
2. gleichzeitig eine ausreichende Stabilität für eine breite Lebensmittelanwendung liefert,
3. sensorisch negative Einflüsse der Johannisbrotfaser auf die Textur von Lebensmitteln minimiert und
4. zur Stabilisierung nach Möglichkeit lösliche Ballaststoffe verwendet und damit eine besonders ernährungsphysiologisch interessante Lebensmittelzutat darstellt.

Gelöst wird diese Aufgabe durch eine Ballaststoffkapsel, deren Kern Johannisbrotfaser enthält und deren Kapselmaterial aus mindestens einem löslichen Ballaststoff und gegebenenfalls stabilisierenden weiteren Materialien besteht. Da bisher zur Anreicherung von Lebensmitteln meist die Ballaststoffe einzeln zugegeben wurden oder bisherige Umhüllungslösungen noch keine ausreichende Stabilitäten in Lebensmitteln zeigten, stellt die im Folgenden beschriebene universelle Stabilisierungsmethode und die daraus resultierenden Stoffe im Vergleich zum Stand der Technik eine erhebliche Verbesserung dar. Dies gilt insbesondere für aufgrund ihrer Farbe und Textur empfindliche Lebensmittel, in welche Johannisbrotfasern nur schwierig einzusetzen sind.

Zusätzlich stellt die vorliegende Erfindung Ballaststoffpräparate zur Verfügung, bei denen verschiedene Effekte von löslichen und unlöslichen Ballaststoffen ideal kombiniert werden. Dadurch lassen sich ideale Lebensmittelzutaten generieren, die zur Anreicherung von Lebensmitteln mit Ballaststoffen oder als funktionelle Zutaten optimal genutzt werden können.

Die erfindungsgemäßen Johannisbrotpräparate dienen als Zutat von Lebensmitteln, Tierfutter oder Arzneimitteln. Dabei können sie der reinen Ballaststoffanreicherung dienen und/oder zur gezielten Zufuhr von funktionellen Zutaten mit einem besonderen Zusatznutzen eingesetzt werden. Weiterhin stellen die erfindungsgemäßen Ballaststoffpräparate Bestandteile dar, die gezielt Produkten zugesetzt werden, welche die menschliche und/oder tierischen Nahrung ergänzen (Nahrungsergänzungsmittel, Dietary Supplements).

Die in den erfindungsgemäßen Präparaten eingesetzte Johannisbrotfaser kann nach bekannten Verfahren aus der Johannisbrotfrucht gewonnen werden. Insbesondere geeignet ist das Verfahren gemäß EP-A-0 616 780. Die so gewonnenen Johannisbrotfasern sind weitgehend wasserunlöslich, d.h. sie enthalten noch max. 5 bis 25 Gew.-%, bevorzugt 10 -15 Gew.-%, wasserlösliche Bestandteile.

Die erfindungsgemäßen Präparate sind dadurch gekennzeichnet, dass die unlösliche Johannisbrotfaser durch eine Hülle allseitig umgeben ist. Als Hüllsubstanzen kommen insbesondere lösliche Ballaststoffe in Frage, die in Tabelle 1 aufgeführt sind. Sie verbessern überraschenderweise die Farbe der Johannisbrotfasern sowie gleichzeitig die geschmacklichen und geruchlichen Eigenschaften. Neben natürlich vorkommenden löslichen Ballaststoffen eignen sich auch fermentativ hergestellte oder chemisch modifizierte Polysaccharide wie sie in Tabelle 1 aufgeführt werden. Zur Erhöhung der Stabilität der Präparate können Hilfsmaterialien verwendet werden. Dafür eignen sich z.B. Emulgatoren, Peptide oder Proteine wie sie ebenfalls in Tabelle 1 angegeben sind.

In den erfindungsgemäßen Präparaten bleiben die ernährungsphysiologisch wünschenswerten Wirkungen des Ballaststoffs wie z.B. auf die Darmperistaltik, auf Blutcholesterinwerte oder die postprandialen Glukosewerte erhalten.

**Tabelle 1:**

| Beispiele für geeignete Kapselmaterialien im Sinne der Erfindung | | | |
|---|---|---|---|
| Lösliche Ballaststoffe, natürlichen Ursprungs | fermentativ gewonnene Polysaccharide | Chemisch modifizierte Polysaccharide | Hilfsmaterialien |
| Gummi Arabicum | Dextrane | Carboxymethyl-cellulose | Lecithin |
| Traganth | Xanthan | Methylcellulose | Tween |
| Karaya Gummi | Curdlan | Hydroxypropyl-cellulose | Albumine |
| Johannisbrotkernmehl | | Hydroxypropyl- | Gelatine |
| Guar Gummi | | methylcellulose | Caseine |
| Alginate | | Niedrig veresterte Pektine | Mineralstoffe |
| Carragenane | | Propylenglykol-alginate | Säurungsmittel |
| Agar-agar | | Methylhydroxyethyl- | Süßungsmittel |
| Psyllium | | cellulose | Aromen |
| β-Glukane | | Ethylhydroxyethyl- | partielle Protein- |
| Inulin | | cellulose | Hydrolysate |
| Oligosaccharide | | Hydrolisiertes Guar | pflanzliche Proteine |
| Maltodextrine | | Gummi | Konservierungs- |
| Pektine | | chemisch modifizierte | stoffe |
| Fenugreekballaststoff | | Stärken | Antioxidantien |
| Arabinogalaktane | | | Pigmente |
| Galaktomannane | | | Farbstoffe |
| Arabinoxylane | | | |

Die verkapselten Johannisbrotfasern sind sphärische oder polygonale Gebilde mit einem - im unverarbeiteten Zustand - mittleren Durchmesser von 1 µm bis 200 µm, bevorzugt 10 bis 100 µm, insbesondere < 70 µm. Im verarbeiteten Zustand - d.h. im Lebensmittel - bleibt der Teilchendurchmesser unverändert, kann aber auch auf das bis zu 5-fache ansteigen.

Der Anteil der unlöslichen Johannisbrotfaser beträgt in Abhängigkeit vom angestrebten Effekt im Produkt, in denen das Ballaststoffpräparat eingesetzt werden soll, 10 bis 90 Gew.-%, bevorzugt 20 bis 50 Gew.-%, insbesondere um 33 Gew.-%. Der Anteil des löslichen, hüllbildenden Ballaststoffes kann je nach Einsatzgebiet von 10 bis zu 90 Gew.-%, bevorzugt 20 bis 75 Gew.-%, insbesondere um 33 Gew.-% liegen. Der Zusatz von Hilfsmaterialien richtet sich nach der zu erzielenden Stabilität und dem gewünschten Effekt und kann zwischen 0-60 Gew.-%, bevorzugt jedoch kleiner 33 Gew.-% liegen. Die o.g. Gew-%-Werte beziehen sich auf die Johannisbrotfaserkapsel.

Zur Herstellung des erfindungsgemäßen Ballaststoffpräparates geht man zweckmäßigerweise so vor, dass man die unlösliche Johannisbrotfaser und die löslichen Ballaststoffe sowie gegebenenfalls die Hilfsmaterialien in ein flüssiges Medium, gewöhnlich ein Lösungs- bzw. Dispersionsmittel, einbringt. Die so erhaltene Suspension wird dann homogen durchmischt und anschließend vom Lösungs- bzw. Dispersionsmittel befreit. Ebenso kann man zunächst eine Lösung eines oder mehrerer löslicher Ballaststoffe und gegebenenfalls eines oder mehreren Hilfsmaterialien vorlegen und darin die unlösliche Johannisbrotfaser homogen suspendieren. Anschließend wird auch hier das Lösungs- bzw. Suspensionsmittel entzogen.

Als flüssiges Medium hat sich Wasser als sehr gut geeignet erwiesen. Des weiteren ist es zweckmäßig, Hilfsmaterialien, die zur Hüllbildung beitragen, mit zu verwenden.

Die Entfernung des Lösungs- bzw. Dispersionsmittels erfolgt durch bekannte Trocknungsverfahren wie z.B. Sprühtrocknung, Wirbelschichttrocknung, Gefriertrocknung u.a., vorzugsweise jedoch durch Sprühtrocknung. Dabei wird normalerweise eine Einstoffdüse zum Versprühen verwendet, die die Entstehung von ausreichend kleinen Partikeln während des Sprühvorgangs gewährleistet. Vorzugsweise werden Düsen mit einem Düsendurchmesser von 0,1 bis 2,0 mm verwendet. Es kann sich aber auch als zweckmäßig erweisen, dass das Hüllmaterial erst unmittelbar beim Verkapseln mit der Mischung zusammenkommen soll, so dass die Zusammenführung im Trockner über eine Zweistoffdüse erfolgt.

Überraschenderweise zeigen die so gewonnenen Johannisbrotfaserkapseln eine im Vergleich zur nicht verkapselten Faser deutlich reduzierte Färbung. Weiterhin werden die aus sensorischer und technologischer Sicht unerwünschten negativen Wahrnehmungen der unlöslichen Johannisbrotfaser deutlich verbessert. Außerdem wurde festgestellt, dass diese Ballaststoffpräparate sich nach dem Trocknungsprozess durch eine hohe Stabilität auszeichnen und daher in empfindlichen Lebensmitteln einsetzbar sind, in denen die unverkapselte Johannisbrotfaser nicht ohne negative Einflüsse auf Farbe und Textur eingesetzt werden können.

Anwendung kann die Erfindung in sehr vielen Lebensmittelgruppen wie Milchprodukten (Joghurts, Quarks, Sauermilchprodukte, Frischkäse, Käsezubereitungen), Backwaren (Brot, Kleingebäck, Konditoreiwaren), Getränken, aber auch in Nahrungsergänzungen, in der Tierernährung (Haus- und Kleintiere; Nutztiere) sowie in kosmetischen Mitteln und in Arzneimitteln u.a. finden.

Im Folgenden wird die Erfindung anhand von Beispielen erläutert.

### Beispiel 1

### Herstellung von verkapselter Johannisbrotfaser:

1 Gew.-% unlösliche Johannisbrotfaser (Caromax™, Nutrinova, Frankfurt, Deutschland) wird in Wasser suspendiert. Das Kapselmaterial, bestehend aus 1 Gew.-% löslichem Ballaststoff Gummi Arabicum und 1 Gew.-% Gelatine wird ebenfalls in Wasser suspendiert. Durch Mischung der beiden Suspensionen wird eine versprühbare Dispersion hergestellt. Diese Dispersion wird während der nachfolgenden Sprühtrocknung zur Gewährleistung einer gleichmäßigen Verteilung der Dispergentien durchgehend mit 500 U min⁻¹ gerührt. Der Trocknungsprozess wurde mit den folgenden Parametereinstellungen durchgeführt:
Trockenlufttemperatur: 170 bis185 °C
Ablufttemperatur: 55 bis 60 °C
Sprühdruck: 1 bar
Ansaugdruck: 0,01 bar

Das resultierende beige-weiße Pulver ist sehr fein und von frei fließendem, partikulärem Charakter.

### Beispiel 2

### Morphologische Änderung beim Verkapseln:

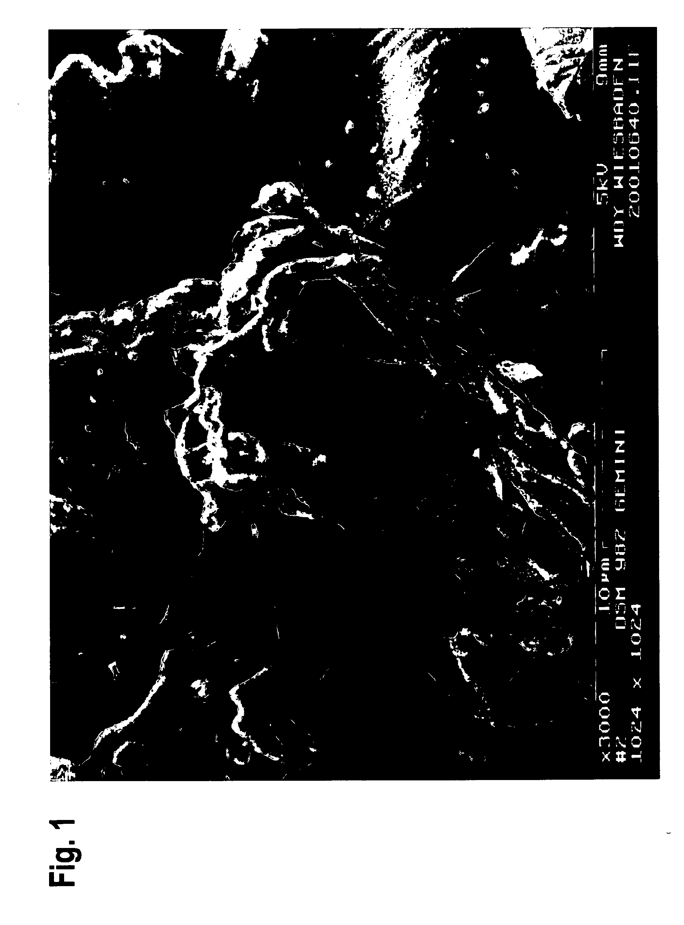

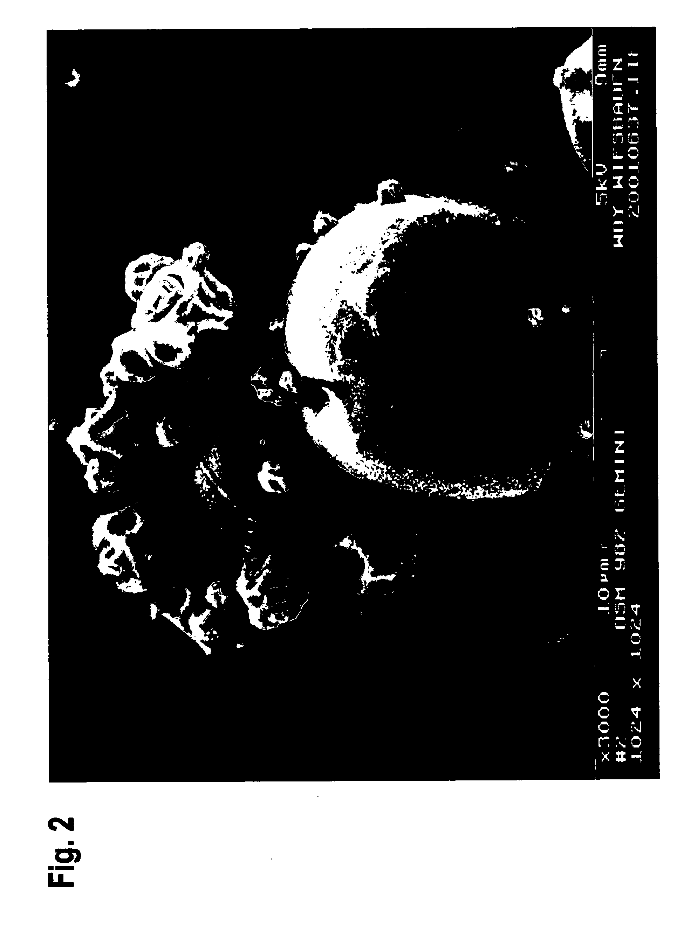

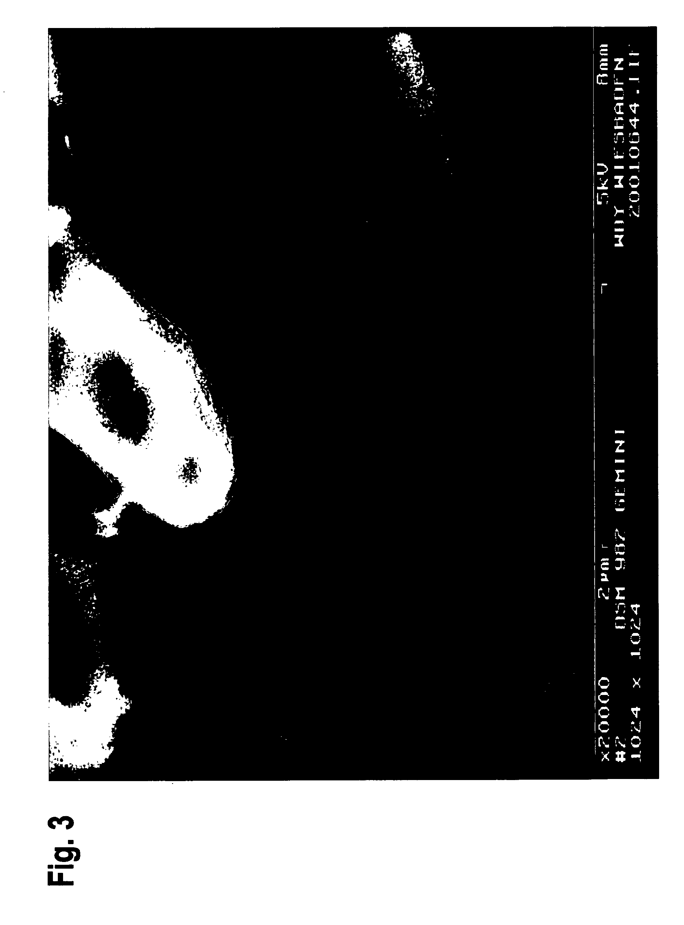

Die nach Beispiel 1 hergestellten verkapselten Johannisbrotfasern wurden elektronenmikroskopisch untersucht. Dabei zeigte sich, dass die ursprünglich irregulär geformten partikulären Johannisbrotfasern (Abb. 1) durch den Verkapselungsprozeß zu gleichmäßig gerundeten Partikeln verändert werden.(Abb. 2). Zusätzlich entstehen sehr kleine (Durchmesser ca. 1 bis 2 µm) Partikel aus reinem Kapselmaterial. Im Anschnittverfahren zeigt sich, dass die Johannisbrotfasern nach dem im Beispiel 1 beschriebenen Verfahren eine Kapseldicke von 1 bis 2 µm aufweisen (Abb. 3).

### Beispiel 3

### Herstellung von verkapselter Johannisbrotfaser:

In 500 g Wasser wurden 9,375g Gelatine eingerührt und unter Erhitzen gelöst. Nach Auflösung und Abkühlung auf ca. 30 °C wurde unter weiterem Rühren 9,375 g Methylhydroxyethylcellulose (MHEC: Marke ®Tylopur MH50G4, Clariant, Wiesbaden, Deutschland) zugegeben und unter weiterem Rühren aufgelöst. In dieser Lösung wurden 6,25 g Johannisbrotfaser (Caromax™) dispergiert. Diese Dispersion wurde während der nachfolgenden Sprühtrocknung zur Gewährleistung einer gleichmäßigen Verteilung der Dispergentien durchgehend mit 500 U min⁻¹ gerührt.
Der Trocknungsprozess wurde mit den folgenden Parametereinstellungen durchgeführt:
Trockenlufttemperatur: 160 °C
Ablufttemperatur: 75 °C
Sprühdruck: 1 bar
Ansaugdruck: 0,01 bar

Das resultierende weißliche, nur leicht beige Pulver ist sehr fein und deutlich heller als das im Beispiel 1 beschriebene Pulver.

### Beispiel 4

### Herstellung eines ballaststoffreichen Joghurts:

In 200 g eines Naturjoghurts wurden 9 g der verkapselten Johannisbrotfasern nach Beispiel 1 eingerührt. Damit ergab sich eine Anreicherung mit 3 Gew.-% Ballaststoff im Endprodukt. Die Farbe des Joghurts wurde durch den Zusatz nur unwesentlich verändert. Die so angereicherten Naturjoghurts zeichneten sich darüber hinaus durch ein deutlich cremigeres, vollmundigeres Mundgefühl im Vergleich zur nicht angereicherten Kontrolle aus. Die Stabilität der verkapselten Faser in diesem Joghurt wurde über einen Zeitraum von mehr als 14 Tagen verfolgt und ergab während der gesamten Zeit keine geschmackliche und optische Veränderung.

### Beispiel 5

### Einsatz in Backwaren (Muffin):

**Tabelle 2:**

| Zutaten zur Herstellung von Muffins | | |
|---|---|---|
| **Zutaten** | **Rezept mit** **verkapselten** **Johannisbrotfasern** | **Referenz-Rezept mit** **Johannisbrotfasern** **(unverkapselt)** |
| | **[g]** | **[g]** |
| Mehl, Typ 405 | 48,65 | 54,10 |
| Backpulver | 1,75 | 1,75 |
| Natron | 0,75 | 0,75 |
| Vollei | 22,5 | 22,50 |
| Zucker | 35,00 | 35,00 |
| Margarine | 31,25 | 31,25 |
| Johannisbrotfasern, verkapselte | 16,45 | ------- |
| Johannisbrotfasern, unverkapselte | --- | 10,90 |
| Buttermilch | 62,5 | 62,5 |
| Σ | 218,75 | 218,75 |
| | | |
| Teigeinwaage | 60 g pro Förmchen | 60 g pro Förmchen |
| Muffinauswaage | 50,2; 50,4; 50,2 Ø 50,3 | 50,4; 50,4; 50,3 Ø 50,4 |
| Backverlust % | 16,17 % | 16,0 % |
| Ballaststoffgehalt % | 5,98 % | 5,93 % |

Verarbeitung: Mehl, (verkapselte) Johannisbrotfaser nach Beispiel 1, Backpulver und Natron mischen. Zucker, Margarine und Vollei schaumig rühren, die Buttermilch und die trockenen Zutaten beifügen und kurz verrühren. Anschließend in Muffinförmchen füllen und bei 160 °C im Umluftherd für 25 min backen.

Eine sensorischer Vergleich der Muffins nach den beiden Rezepten ergab (Tabelle 3), dass die mit verkapselter Johannisbrotfaser gebackenen Muffins deutlich besser als die mit reiner Johannisbrotfaser gebackenen beurteilt wurden.

**Tabelle 3:**

| Sensorische Bewertung von Backwaren | | |
|---|---|---|
| **Beurteilung:** | **Rezept mit verkapselten** **Johannisbrotfasern** | **Referenz-Rezept mit** **Johannisbrotfasern** **(unverkapselt)** |
| Aussehen/Farbe außen: | hellbraun | schokobraun |
| innen: | beige-braun | schokobraun |
| | feine Porung ähnlich wie bei | feine Porung ähnlich wie bei |
| | Rührkuchen | Rührkuchen |
| Geruch | typisch nach Ei, | deutlich nach Johannisbrot, malzig, |
| | leicht nach Johannisbrot | brandig |
| Geschmack | am Anfang neutral, später leicht | deutlich nach Johannisbrot, malzig |
| | nach Johannisbrot, leicht malzig; | |
| Textur | sehr locker, feucht, Partikel kaum spürbar | sehr locker, feucht, Partikel spürbar |
| Gesamteindruck | gut | akzeptabel |

## Patentansprüche

1. Ballaststoffkapsel, bestehend aus einem Kern aus im wesentlichen wasserunlöslicher Johannisbrotfaser und einem Kapselmaterial aus mindestens einem wasserlöslichen Ballaststoff.

2. Ballaststoffkapsel nach Anspruch 1, **dadurch gekennzeichnet, dass** das Kapselmaterial noch mindestens ein Hilfsmaterial enthält.

3. Ballaststoffkapsel nach Anspruch 1 oder 2, **dadurch gekennzeichnet, dass** der wasserlösliche Ballaststoff ausgewählt wird aus einer oder mehreren der folgenden Substanzgruppen: wasserlösliche Ballaststoffe natürlichen Ursprungs, fermentativ gewonnene Polysaccharide und chemisch modifizierte Polysaccharide.

4. Ballaststoffkapsel nach Anspruch 3, **dadurch gekennzeichnet, dass** derwasserlösliche Ballaststoff natürlichen Ursprungs ausgewählt wird aus einer oder mehreren der folgenden Substanzen: Gummi Arabicum, Traganth, Karaya Gummi, Johannisbrotkernmehl, Guar Gummi, Alginaten, Carragenanen, Agar-agar, Psyllium, β-Glukanen, Inulin, Oligosaccariden, Maltodextrinen, Pektinen, Fenugreekballaststoff, Arabinogalaktanen, Galaktomannane und Arabinoxylane.

5. Ballaststoffkapsel nach Anspruch 3, **dadurch gekennzeichnet, dass** das fermentativ gewonnene Polysaccharid ausgewählt wird aus einer oder mehreren der folgenden Substanzen: Dextranen, Xanthan und Curdlan.

6. Ballaststoffkapsel nach Anspruch 3, **dadurch gekennzeichnet, dass** das chemisch modifizierte Polysaccharid ausgewählt wird aus einer oder mehreren der folgenden Substanzen: Carboxymethylcellulose, Methylcellulose, Hydroxypropylcellulose, Hydroxypropylmethylcellulose, niedrig veresterten Pektinen, Propylenglykolalginaten, Methylhydroxyethylcellulose, Ethylhydroxyethylcellulose, hydrolisiertes Guar Gummi und chemisch modifizierte Stärken.

7. Ballaststoffkapsel nach Anspruch 2, **dadurch gekennzeichnet, dass** das Hilfsmaterial ausgewählt wird aus einer oder mehreren der folgenden Substanzen: Lecithin, Tween, Albuminen, Gelatine, Caseinen, Mineralstoffen, Säurungsmitteln, Süßungsmitteln, Aromen, partielle Protein-Hydrolysate, pflanzliche Proteine, Konservierungsstoffe, Antioxidantien, Pigmente und Farbstoffe.

8. Ballaststoffkapsel nach einem der Ansprüche 1 bis 7, **dadurch gekennzeichnet, dass** die Johannisbrotfaser in der Kapsel in einer Menge von 10 bis 90 Gew.-% (bezogen auf die Kapsel) enthalten ist.

9. Ballaststoffkapsel nach einem der Ansprüche 1 bis 8, **dadurch gekennzeichnet, dass** der wasserlösliche Ballaststoff in der Kapsel in einer Menge von 10 bis 90 Gew.-% (bezogen auf die Kapsel) enthalten ist.

10. Ballaststoffkapsel nach einem der Ansprüche 2 bis 9, **dadurch gekennzeichnet, dass** das Hilfsmaterial in der Kapsel in einer Menge von >0 bis 60 Gew.-% (bezogen auf die Kapsel) enthalten ist.

11. Ballaststoffkapsel nach einem der Ansprüche 1 bis 10, **dadurch gekennzeichnet, dass** sie sphärische oder polygonale Gestalt hat.

12. Ballaststoffkapsel nach Anspruch 11, **dadurch gekennzeichnet, dass** sie - im unverarbeiteten Zustand - einen mittleren Durchmesser von 1 bis 200 µm besitzt.

13. Verfahren zur Herstellung einer Ballaststoffkapsel nach Anspruch 1, **dadurch gekennzeichnet, dass** man die Johannisbrotfaser und den wasserlöslichen Ballaststoff in einem flüssigen Medium suspendiert und anschließend das flüssige Medium ganz oder teilweise entzieht.

14. Verfahren nach Anspruch 13, **dadurch gekennzeichnet, dass** das Entziehen des flüssigen Mediums durch Sprühtrocknung erfolgt.

15. Verwendung einer Ballaststoffkapsel nach Anspruch 1 in Lebensmitteln oder Nahrungsergänzungsmitteln.

16. Verwendung einer Ballaststoffkapsel nach Anspruch 1 in Tiernahrung.

17. Verwendung einer Ballaststoffkapsel nach Anspruch 1 in Arzneien.

18. Verwendung einer Ballaststoffkapsel nach Anspruch 1 in Kosmetika.
